# EUROPEAN PATENT SPECIFICATION

(11) **EP 4 126 801 B1**
(45) Date of publication and mention of the grant of the patent: **06.05.2026**
(21) Application number: 21719186.5
(22) Date of filing: 30.03.2021
(51) Int. Cl.: C07C 29/80, C07C 31/20

(54) **RECOVERING MONO-PROPYLENE GLYCOL**
RÜCKGEWINNUNG VON MONOPROPYLENGLYKOL
RÉCUPÉRATION DE MONO-PROPYLÈNE GLYCOL

(30) Priority: 03.04.2020 FI 20205345
(43) Date of publication of application: 08.02.2023
(73) Proprietor: UPM-Kymmene Corporation, 00100 Helsinki (FI)
(72) Inventor: KAJANTO, Isko, 02200 Espoo (FI)
(74) Representative: Papula Oy
(86) International application number: PCT/FI2021/050230
(87) International publication number: WO 2021/198563

(56) References cited:
- WO-A1-2004/076392
- WO-A1-2017/202727
- CN-A- 106 117 013
- US-A1- 2017 327 446
- VANDEKERKHOVE ANNELIES ET AL: "Rh-Catalyzed Hydrogenation of Amino Acids to Biobased Amino Alcohols: Tackling Challenging Substrates and Application to Protein Hydrolysates", ACS SUSTAINABLE CHEMISTRY & ENGINEERING, vol. 6, no. 7, 1 June 2018 (2018-06-01), US, pages 9218 - 9228, XP093331216, ISSN: 2168-0485, DOI: 10.1021/acssuschemeng.8b01546

## Description

### TECHNICAL FIELD

The present disclosure relates to a method for recovering mono-propylene glycol from a mixture comprising bio-derived diols. The present disclosure further relates to mono-propylene glycol. The invention is defined in the claims.

### BACKGROUND

Mono-propylene glycol (MPG, also called 1,2-propanediol), is an important raw material finding use e.g. in the manufacturing of polymers. Mono-propylene glycol is a compound which is generally recognized as safe and can be further used for e.g. food applications as well as a vehicle for topical, oral and some intravenous pharmaceutical preparations. Mono-propylene glycol can be produced from propylene oxide e.g. by a non-catalytic high-temperature process at 200 °C - 220 °C, or by a catalytic process, which proceeds at 150 °C - 180 °C in the presence of ion exchange resin or a small amount of sulfuric acid or alkali. Mono-propylene glycol can also be obtained from glycerol, a byproduct from the production of bio-diesel.

In addition, mono-propylene glycol may be produced from sugars together with mono-ethylene glycol. However, when producing such polyols as mono-ethylene glycol and mono-propylene glycol from sugars also other diols, alcohols and other substances are formed as side-products. Typically, when mono-ethylene glycol is distilled from such a composition, mono-propylene glycol may be obtained as a side-product together with other lighter impurities and needs further purification. The purification of the mono-propylene glycol has however been challenging. The inventor has thus recognized the need to provide a manner for recovering purified mono-propylene glycol e.g. from the side-product when producing mono-ethylene glycol.

WO 2017/202727 A1 discloses a process for the production of a high purity first diol from a product stream comprising two or more C2 to C7 diols.

### SUMMARY

A method for recovering mono-propylene glycol from a mixture comprising bio-derived diols is disclosed. The mixture may comprise an organic impurity. The method may comprise: (ia) separating the organic impurity from mono-propylene glycol in a first distillation process. The first distillation process may be carried out at a temperature within the range of 140 - 200 °C and a pressure within the range of 0.3 - 1.0 bar. The method may further comprise (ii) recovering mono-propylene glycol.

Further is disclosed a method for recovering mono-propylene glycol from a mixture comprising bio-derived diols and an organic impurity, wherein the mixture comprises mono-propylene glycol in an amount of at least 50 weight-% of the total weight of the mixture. The method may comprises:
(ia) separating the organic impurity from mono-propylene glycol in a first distillation process, wherein the first distillation process is carried out at a temperature within the range of 140 - 200 °C and a pressure within the range of 0.3 - 1.0 bar; and
(ib) separating diols that have a boiling point lower than the boiling point of mono-propylene glycol, from mono-propylene glycol in a second distillation process, wherein the second distillation process is carried out at a temperature within the range of 90 - 150 °C and a pressure within the range of 0.05 - 0.2 bar. The method may further comprise (ii) recovering mono-propylene glycol.

Further it is disclosed mono-propylene glycol obtainable by the method as disclosed in the current specification.

### BRIEF DESCRIPTION OF THE DRAWINGS

The accompanying drawings, which are included to provide a further understanding of the embodiments and constitute a part of this specification, illustrate various embodiments. In the drawings:
Fig. 1a discloses one embodiment of the arrangement disclosed in the current specification;
Fig. 2a discloses one embodiment of the arrangement disclosed in the current specification; and
Fig. 2b discloses another embodiment of the arrangement disclosed in the current specification.

### DETAILED DESCRIPTION

A method for recovering mono-propylene glycol from a mixture comprising bio-derived diols is disclosed. The mixture may comprise an organic impurity. The method may comprise: (ia) separating the organic impurity from mono-propylene glycol in a first distillation process. The first distillation process may be carried out at a temperature within the range of 140 - 200 °C and a pressure within the range of 0.3 - 1.0 bar. The method may further comprise (ii) recovering mono-propylene glycol.

Further is disclosed a method for recovering mono-propylene glycol from a mixture comprising bio-derived diols and an organic impurity, wherein the mixture comprises mono-propylene glycol in an amount of at least 50 weight-% of the total weight of the mixture. The method may comprises:
(ia) separating the organic impurity from mono-propylene glycol in a first distillation process, wherein the first distillation process is carried out at a temperature within the range of 140 - 200 °C and a pressure within the range of 0.3 - 1.0 bar; and
(ib) separating diols that have a boiling point lower than the boiling point of mono-propylene glycol, from mono-propylene glycol in a second distillation process, wherein the second distillation process is carried out at a temperature within the range of 90 - 150 °C and a pressure within the range of 0.05 - 0.2 bar. The method may further comprise (ii) recovering mono-propylene glycol.

Further is disclosed mono-propylene glycol obtainable by the method as disclosed in the current specification.

The method for recovering mono-propylene glycol may be carried out by using an arrangement for recovering mono-propylene glycol from a mixture comprising bio-derived diols. The arrangement may comprise: a first distillation column configured to operate at a temperature within the range of 140 - 200 °C and a pressure within the range of 0.3 - 1.0 bar for separating the organic impurity from mono-propylene glycol. The arrangement may further comprise a recovering unit configured to recover mono-propylene glycol.

In this specification, the term "temperature" within a specified range, is used to refer to the temperature that is used to carry out the distillation process as such or the temperature that is used in the distillation column, respectively. It is clear to the person skilled in the art that the temperature in the distillation column as such may differ from the temperature in e.g. the condenser or the reboiler that may be operationally connected to the distillation column. The term "temperature" within a specified range, may be the temperature at any part of distillation column as such.

In this specification, the term "pressure" within a specified range, is used to refer to the top pressure of the distillation process or the distillation column, respectively.

Distillation may generally be considered a process of separating components or substances from a liquid mixture by using selective boiling and condensation. Distillation may result in essentially complete separation into nearly pure components, or it may be a partial separation that increases the concentration of selected components in the mixture. The distillation process exploits differences in the relative volatility of the different components in the mixture.

The mixture comprising bio-derived diols may comprise e.g. mono-ethylene glycol (MEG, also called ethylene glycol or 1,2-ethanediol), mono-propylene glycol (MPG, also called 1,2-propanediol), butylene glycol (BDO, also called butanediol), and an organic impurity. Such a mixture of bio-based diols may be derived e.g. from a process for the production of glycols, such as a process for producing mono-ethylene glycol. In one embodiment, the mixture comprising bio-derived diols comprises mono-propylene glycol, butylene glycols and the organic impurity. Butylene glycol may appear in structures differing from each other in where the OH-units are situated. Such structures are e.g. 1,2-butanediol, 2,3-butanediol, and 1,4-butanediol.

The mixture may comprise mono-ethylene glycol, mono-propylene glycol, butylene glycol, and an organic impurity in an amount of at least 80 weight-%, or at least 85 weight-%, or at least 90 weight-%, or at least 92 weight-%, based on the total weight of the mixture. The method may comprise recovering mono-propylene glycol from a mixture that comprises mono-ethylene glycol, mono-propylene glycol, butylene glycol, and an organic impurity in an amount of at least 80 weight-%, or at least 85 weight-%, or at least 90 weight-%, or at least 92 weight-%, based on the total weight of the mixture. The mixture may comprise mono-propylene glycol, butylene glycol, and an organic impurity in an amount of at least 80 weight-%, or at least 85 weight-%, or at least 90 weight-%, or at least 92 weight-%, based on the total weight of the mixture. The method may comprise recovering mono-propylene glycol from a mixture that comprises mono-propylene glycol, butylene glycol, and an organic impurity in an amount of at least 80 weight-%, or at least 85 weight-%, or at least 90 weight-%, or at least 92 weight-%, based on the total weight of the mixture.

In one embodiment, the mixture comprises mono-propylene glycol in an amount of at least 50 weight-%, or at least 60 weight-%, or at least 70 weight-%, or at least 80 weight-%, or at least 90 weight-%, based on the total weight of the mixture. The method may comprise recovering mono-propylene glycol from a mixture that comprises mono-propylene glycol in an amount of at least 50 weight-%, or at least 60 weight-%, or at least 70 weight-%, or at least 80 weight-%, or at least 90 weight-%, based on the total weight of the mixture.

The mixture comprising bio-derived diols may further comprise water. In one embodiment, the mixture comprises water in an amount of 5 - 10 weight-%, or 7 - 8 weight-%, based on the total weight of the mixture. In one embodiment, the mixture comprises essentially no water.

As above presented, mono-propylene glycol may be produced as a side-product from a process to prepare a liquid composition of glycols comprising e.g. mono-ethylene glycol.

Such a liquid composition of glycols may be prepared from wood-based raw material, such as from hardwood or softwood. The wood-based raw material may originate from e.g. pine, poplar, beech, aspen, spruce, eucalyptus, ash, oak, maple, chestnut, willow, or birch. The wood-based raw material may also be any combination or mixture of these.

In one embodiment, a method for producing a liquid composition of glycols may comprise:
- providing a wood-based feedstock originating from wood-based raw material and comprising wood chips, and subjecting the wood-based feedstock to at least one pretreatment to form a liquid fraction and a fraction comprising solid cellulose particles;
- subjecting the fraction comprising solid cellulose particles to enzymatic hydrolysis to form a lignin fraction and a carbohydrate fraction;
- subjecting the carbohydrate fraction to catalytical conversion to form a liquid composition of glycols.

Providing the wood-based feedstock may comprise subjecting wood-based raw material to a mechanical treatment selected from debarking, chipping, dividing, cutting, beating, grinding, crushing, splitting, screening, and/or washing the wood-based raw material to form the wood-based feedstock. Providing the wood-based feedstock may comprise purchasing the wood-based feedstock.

Pretreatment of the wood-based feedstock may comprise at least one of the following: pre-steaming of the wood-based feedstock, subjecting the wood-based feedstock to an impregnation treatment, and subjecting the wood-based feedstock to steam explosion.

The pretreatment may comprise subjecting the wood-based feedstock to pre-steaming. The pretreatment may comprise, an impregnation treatment and/or a steam explosion and may comprise, before subjecting the wood-based feedstock to impregnation treatment and/or to steam explosion, subjecting the wood-based feedstock to pre-steaming. The pre-steaming of the wood-based feedstock may be carried out with steam having a temperature of 100 - 130 °C at atmospheric pressure. During the pre-steaming the wood-based feedstock is treated with steam of low pressure. The pre-steaming may be also carried out with steam having a temperature of below 100 °C, or below 98 °C, or below 95 °C.

Further, the pretreatment may comprise subjecting the wood-based feedstock to at least one impregnation treatment with an impregnation liquid. The impregnation treatment may be carried out to the wood-based feedstock received from the mechanical treatment and/or from the pre-steaming. The pretreatment may comprise, before subjecting to the steam explosion, subjecting the wood-based feedstock to at least one impregnation treatment with an impregnation liquid selected from water, at least one acid, at least one alkali, at least one alcohol, or any combination or mixture thereof. The impregnation liquid may comprise water, at least one acid, at least one alkali, at least one alcohol, or any combination or mixture thereof.

The pretreatment may comprise subjecting the wood-based feedstock to steam explosion. The wood-based feedstock from the mechanical treatment, the pre-steaming step, and/or from the impregnation treatment may be subjected to steam explosion.

The pretreatment may comprise at least one of mechanical treatment of wood-based material to form wood-based feedstock, pre-steaming of the wood-based feedstock, impregnation treatment of the wood-based feedstock, and steam explosion of the wood-based feedstock. The pretreatment may comprise mechanical treatment of wood-based material to form a wood-based feedstock, pre-steaming of the wood-based feedstock, impregnation treatment of the pre-steamed wood-based feedstock, and steam explosion of the impregnated wood-based feedstock. The pretreatment may comprise pre-steaming the wood-based feedstock, impregnation treatment of the pre-steamed wood-based feedstock, and steam explosion of the impregnated wood-based feedstock. The pretreatment may comprise impregnation treatment of the wood-based feedstock, and steam explosion of the impregnated wood-based feedstock. I.e. the wood-based feedstock having been subjected to the impregnation treatment may thereafter be subjected to the steam explosion. Also, the wood-based feedstock having been subjected to pre-steaming, may then be subjected to the impregnation treatment and thereafter the wood-based feedstock having been subjected to the impregnation treatment may be subjected to steam explosion.

In this specification, the term "steam explosion" may refer to a process of hemihydrolysis in which the wood-based feedstock is treated in a reactor with steam having a temperature of 130 - 240 °C under a pressure of 0.17 - 3.25 MPaG followed by a sudden, explosive decompression of the steam-treated wood-based feedstock that results in the rupture of the fiber structure. The output from the steam explosion may be mixed with a suitable liquid, e.g. water, to form a slurry comprising solid cellulose particles. The fraction comprising solid cellulose particles may be separated from the liquid fraction by a suitable separation method, e.g. by a solid-liquid separation.

The enzymatic hydrolysis of the fraction comprising solid cellulose particles may be carried out at a temperature of 30 - 70 °C, or 35 - 65 °C, or 40 - 60 °C, or 45 - 55 °C, or 48 - 53 °C while keeping the pH of the fraction comprising solid cellulose particles at a pH value of 3.5 - 6.5, or 4.0 - 6.0, or 4.5 - 5.5, and wherein the enzymatic hydrolysis is allowed to continue for 20 - 120 h, or 30 - 90 h, or 40 - 80 h. Enzymatic hydrolysis may result in the formation of a lignin fraction and a carbohydrate fraction. The enzymes are catalysts for the enzymatic hydrolysis. The enzymatic reaction decreases the pH and by shortening the length of the cellulose fibers it may also decrease the viscosity. Subjecting the fraction comprising solid cellulose particles to enzymatic hydrolysis may result in cellulose being transformed into glucose monomers with enzymes. Lignin present in the fraction comprising solid cellulose particles may remain essentially in solid form.

At least one enzyme may be used for carrying out the enzymatic hydrolysis. The at least one enzyme may be selected from a group consisting of cellulases, hemicellulases, laccases, and lignolytic peroxidases. Cellulases are multi-protein complexes consisting of synergistic enzymes with different specific activities that can be divided into exo- and endo-cellulases (glucanase) and β-glucosidase (cellobiose). The enzymes may be either commercially available cellulase mixes or on-site manufactured.

Catalytical conversion of the carbohydrate fraction may comprise subjecting the carbohydrate fraction to catalytical hydrogenolysis. I.e. the carbohydrate fraction may be subjected to catalysts in the presence of hydrogen in step iii). The catalytical conversion may be carried out in the presence of water. In one embodiment, the catalytical conversion of the carbohydrate fraction comprises subjecting the carbohydrate fraction to catalytical hydrogenation in the presence of a solvent, preferably water and a catalyst system. The catalytical conversion may be carried out in the presence of a catalyst system comprising one or more catalysts.

Subjecting the carbohydrate fraction to catalytical conversion may result in a liquid composition of glycols. The catalytical conversion accomplishes at least hydrogenolation and hydrocracking reactions to achieve hydrogenolation and hydrocracking of the carbohydrate fraction such that a liquid composition of glycols is formed. The liquid composition of glycols may comprise or consist of mono-ethylene glycol, mono-propylene glycol and butylene glycol. These glycols may be present at a concentration of 0.1 - 40 weight-% based on the total weight of the liquid composition of glycols. The liquid composition of glycols may also comprise other side products.

E.g. mono-ethylene glycol may be recovered from the liquid composition of glycols e.g. by a separation technique selected form adsorption, evaporation, distillation, extractive distillation, azeotrope distillation, vacuum distillation, atmospheric distillation, membrane separation, filtration, reactive purification or a combination of them.

When most-part of the mono-ethylene glycol is recovered from the liquid composition of glycols, the mixture comprising bio-derived diols may simultaneously be recovered as a side-product. However, some part of the mono-ethylene glycol of the liquid composition of glycols may remain in the mixture.

The mixture comprising bio-derived diols applied in the current specification may however also be provided from any other process for the production of glycols. The method as described in the current specification should not be understood to be bound to the above described process for producing a liquid composition of glycols.

By the expression "mixture comprising bio-derived diols" should be understood in this specification, unless otherwise stated, as a mixture of one or more diols, which are derived from a bio-based origin or raw material. In one embodiment, the bio-derived diols are wood-derived diols. The diols may thus be derived from e.g. hardwood, softwood, or from a combination of these. The diols may also be derived from broadleaf wood. The diols may be derived e.g. from pine, poplar, beech, aspen, spruce, eucalyptus, ash, or birch, or from any combination or mixture of these.

The inventor found out that the mixture comprising bio-derived diols may also comprise an organic impurity. In one embodiment, the organic impurity is characterized by a retention time of 6.5 - 6.7 minutes when determined by gas-chromatography-flame ionization detector (GC-FID). In one embodiment, the organic impurity is characterized by a retention time of 6.5 - 6.7 minutes when determined by a gas-chromatography-flame ionization detector (GC-FID) with the following parameters: The column is DB-HeavyWax (30 m x 0.32 mm, 0.5 µm); the carrier gas is helium at a flow rate of 1.9 ml/min; injection temperature is 250 °C. Samples are injected without dilution for identification or qualitative analysis. The starting temperature is 140 °C and the oven is kept at this temperature for 10 minutes. Then the temperature is raised to 270 °C at a heating rate of 15 °C per minute. Then the sample is kept at this temperature for 10 minutes. The total operation time is 28.67 min.

In one embodiment, the organic impurity is characterized by the tallest peak value at 59 m/z when determined by gas-chromatography-mass-spectrometer (GC-MS). In one embodiment, the organic impurity is characterized by the tallest peak value at 59 m/z when determined by gas-chromatography-mass-spectrometer (GC-MS) with the above mentioned column. The organic impurity may be further characterized by an additional peak value at 45 m/z when determined by gas-chromatography-mass-spectrometer (GC-MS).

The organic impurity may form an azeotrope with mono-propylene glycol, whereby separating it from mono-propylene glycol in order to get a high yield of pure mono-propylene glycol may be challenging. The inventor found out that the azeotrope may exist at low pressures but when the pressure is raised to at least 0.3 bar, e.g. to at least 0.5 bar or at least 0.7 bar, the azeotrope may disappear or it may be broken, such that the organic impurity and mono-propylene glycol may be at least partly separated by distillation.

Thus, the method comprises separating the organic impurity from mono-propylene glycol in a first distillation process, wherein the first distillation process is carried out at a temperature within the range of 140 - 200 °C and a pressure within the range of 0.3 - 1.0 bar. In one embodiment, the first distillation process is carried out at a temperature within the range of 150 - 190 °C, or 160 - 180 °C. In one embodiment, the first distillation process is carried out at a pressure within the range of 0.5 - 0.9 bar, or 0.7 - 0.8 bar.

The first distillation process may be carried out in a first distillation column. The first distillation column may be configured to operate at a temperature within the range of 140 - 200 °C and a pressure within the range of 0.3 - 1.0 bar for separating the organic impurity from mono-propylene glycol. In one embodiment, the first distillation column is configured to operate at a temperature within the range of 150 - 190 °C, or 160 - 180 °C. In one embodiment, the first distillation column is configured to operate at a pressure within the range of 0.5 - 0.9 bar, or 0.7 - 0.8 bar.

The method may comprise feeding the mixture into the first distillation process. The arrangement may comprise a pump configured to feed the mixture into the first distillation column.

The method as disclosed in the current specification may further comprise: (ib) separating diols that have a boiling point lower than the boiling point of mono-propylene glycol, from mono-propylene glycol in a second distillation process, wherein the second distillation process is carried out at a temperature within the range of 90 - 150 °C and a pressure within the range of 0.05 - 0.2 bar. 2,3-butanediol may be mentioned as one example of a diol that may be separated from mono-propylene glycol in step (ib). Also other components that are possibly present and have a boiling point lower than the boiling point of mono-propylene glycol may be separated from mono-propylene glycol in step (ib).

Steps (ia) and (ib) may be are carried out one after the other in any order. I.e. step (ia) may precede step (ib), or vice versa. In one embodiment, (ia) is carried out before (ib), or (ia) is carried out after (ib). In one embodiment, steps (ia) and (ib) are sequential steps carried out one after the other in any order. I.e. step (ia) may be directly followed by step (ib) or step (ib) may be directly followed by step (ia).

In a corresponding manner, the arrangement may comprise a second distillation column configured to operate at a temperature within the range of 90 - 150 °C and a pressure within the range of 0.05 - 0.2 bar for separating diols that have a boiling point lower than the boiling point of mono-propylene glycol, from mono-propylene glycol. The first distillation column and the second distillation column may be operationally arranged one after the other in any order. I.e. the first distillation column may be operationally arranged before the second distillation column, or vice versa.

In one embodiment, the first distillation column is operationally arranged before the second distillation column or the first distillation column is operationally arranged after the second distillation column. In one embodiment, the first distillation column is operationally arranged before the second distillation column. In one embodiment, the first distillation column is operationally arranged after the second distillation column.

In one embodiment, the second distillation process is carried out at a temperature within the range of 100 - 140 °C, or 110 - 130 °C. In one embodiment, the second distillation process is carried out at a pressure within the range of 0.1 - 0.15 bar. In one embodiment, the second distillation process is carried out at a pressure within the range of 0.05 - 0.15 bar or 0.1 - 0.2 bar. In one embodiment, the second distillation column is configured to operate at a temperature within the range of 100 - 140 °C, or 110 - 130 °C. In one embodiment, the second distillation column is configured to operate at a pressure within the range of 0.1 - 0.15 bar. In one embodiment, the second distillation column is configured to operate at a pressure within the range of 0.05 - 0.15 bar or 0.1 - 0.2 bar.

The method as disclosed in the current specification may further comprise:
- removing the organic impurity in a first bottom stream from the first distillation process; and
- removing mono-propylene glycol in a first top stream from the first distillation process.

The method as disclosed in the current specification may further comprise:
- removing diols that have a boiling point lower than the boiling point of mono-propylene glycol in a second top stream from the second distillation process; and
- removing mono-propylene glycol in a second bottom stream from the second distillation process.

In one embodiment, the first distillation process is carried out before the second distillation process. In one embodiment, the first top stream may comprise, in addition to mono-propylene glycol, diols that have a boiling point lower than the boiling point of mono-propylene glycol. In one embodiment, the method comprises feeding the first top stream into the second distillation process. In one embodiment, the arrangement comprises a pump configured to feed the first top stream into the second distillation column. The first bottom stream may comprise the organic impurity.

In one embodiment, the first distillation process is carried out after the second distillation process. In one embodiment the second bottom stream comprises, in addition to mono-propylene glycol, the organic impurity. In one embodiment, the method comprises feeding the second bottom stream into the first distillation process. In one embodiment, the arrangement comprises a pump configured to feed the second bottom stream into the first distillation column.

Diols that have a boiling point lower than the boiling point of mono-propylene glycol may be separated from mono-propylene glycol in the distillation conditions provided in the second distillation column. 2,3-butanediol can be mentioned as an example of such diols. 2,3-butanediol has a boiling point of 177 °C in atmospheric pressure, while the boiling point of mono-propylene glycol is 187 °C in atmospheric pressure.

The second top stream from the second distillation process may not essentially contain mono-propylene glycol. The second top stream may contain butylene glycol, such as 2,3-butylene glycol. The second top stream may also comprise water.

Thus, as a result of the second distillation process, the mono-propylene glycol having a higher boiling point than e.g. other diols possibly present in the mixture, may be removed or discharged from the second distillation process in a second bottom stream.

The mono-propylene glycol may be recovered from the second distillation process in the second bottom stream. The second bottom stream may in addition to mono-propylene glycol comprise a minor or residual amount of mono-ethylene glycol and/or butylene glycol. The amount of mono-ethylene glycol in the second bottom stream may be at most 0.5 weight-%, or at most 0.3 weight-%, or at most 0.2 weight-%, based on the total weight of the second bottom stream. The amount of butylene glycol in the second bottom stream may be at most 0.1 weight-%, or at most 0.08 weight-%, or at most 0.07 weight-%, based on the total weight of the second bottom stream.

If the second distillation process is carried out before the first distillation process, then the second bottom stream may in addition to the mono-propylene glycol comprise the organic impurity.

If the second distillation process is carried out after the first distillation process, then the second bottom stream may not contain organic impurity in an essential amount, as the organic impurity may have been separated from the mono-propylene glycol in the first distillation process.

Thus, the first distillation process has the added utility of enabling separation of the organic impurity and mono-propylene glycol such that mono-propylene glycol can be recovered in a high concentration. The inventor surprisingly found out that by the operating conditions used in the first distillation process, it is possible to break up the azeotrope that may be formed between the organic impurity and mono-propylene glycol. The mono-propylene glycol may be recovered from the first distillation process as an first top stream, whereas the organic impurity may be recovered from the first distillation process as a first bottom stream.

The first distillation column may comprise 50 - 120 distillation stages. The second distillation column may comprise 50 - 120 distillation stages.

In one embodiment, the reflux ratio is at least 5, or at least 10.

The arrangement may also comprise a reboiler and/or a condenser. I.e. a distillation column may be operationally connected to a reboiler and/or a condenser.

The method may comprise recovering mono-propylene glycol at a concentration of at least 99 weight-%, or at least 99.5 weight-%, or at least 99.7 weight-%, or at least 99.9 weight-%, or at least 99.99 weight-%. Disclosed is mono-propylene glycol obtainable by the method as disclosed in the current specification, wherein mono-propylene glycol is recovered at a concentration of at least 99 weight-%. In on embodiment is disclosed mono-propylene glycol, wherein the mono-propylene glycol is recovered at a concentration of at least 99.5 weight-%, or at least 99.7 weight-%, or at least 99.9 weight-%, or at least 99.99 weight-%.

The arrangement may comprise a recovering unit configured to recover mono-propylene glycol. In one embodiment, the recovering unit is a tank, a storage bin or the like.

The method as described in the current specification has the added utility of enabling to separate the organic impurity present from mono-propylene glycol. The method as described in the current specification has the added utility of enabling to recover mono-propylene glycol from a stream of bio-based diols at a concentration of up to e.g. 99.9 %. The use of the first distillation process has the added utility of enabling the use of such distillation conditions that the possible azeotrope between the organic impurity and mono-propylene glycol may disappear and the separation of the organic impurity and mono-propylene glycol is possible.

### EXAMPLES

Reference will now be made in detail to various embodiments.

The description below discloses some embodiments in such a detail that a person skilled in the art is able to utilize the embodiments based on the disclosure. Not all steps or features of the embodiments are discussed in detail, as many of the steps or features will be obvious for the person skilled in the art based on this specification.

For reasons of simplicity, item numbers will be maintained in the following exemplary embodiments in the case of repeating components.

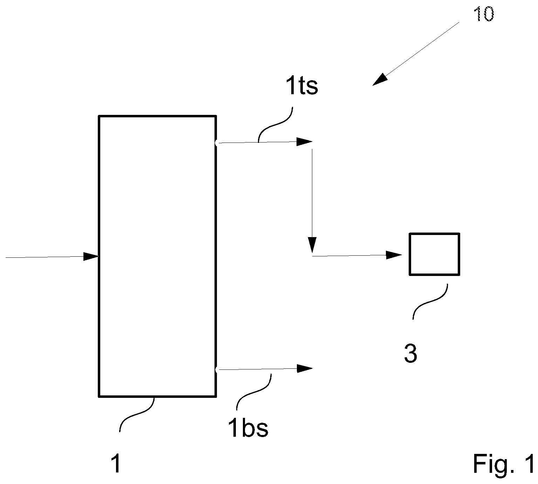

The enclosed Fig. 1 discloses an example of an embodiment of the method for recovering mono-propylene glycol from a mixture comprising bio-derived diols carried out in an corresponding arrangement. Fig. 1 discloses an embodiment comprising a first distillation column 1. In the embodiment of Fig. 1 a mixture comprising bio-derived diols may be fed into the first distillation column, wherein the first distillation process is carried out. The mixture may comprise mono-propylene glycol and the organic impurity. The first distillation column 1 may be configured to operate at a temperature within the range of 140 - 200 °C and a pressure within the range of 0.3 - 1.0 bar. As a result of the first distillation process the organic impurity may be removed in a first bottom stream 1bs from the first distillation column 1 and the mono-propylene glycol may be removed in a first top stream 1ts from the first distillation column 1.

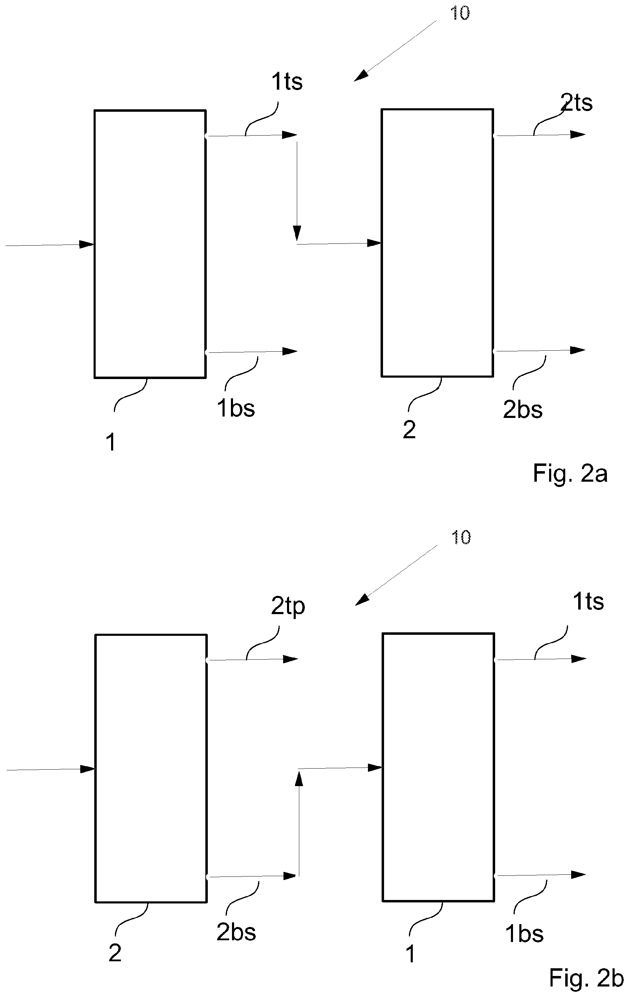

The enclosed Fig. 2a and Fig. 2b illustrate examples of an embodiment of the method for recovering mono-propylene glycol from a mixture comprising bio-derived diols carried out in corresponding arrangements 10.

Both Fig. 2a and Fig. 2b discloses embodiments of arrangements 10 comprising a first distillation column 1 for separating the organic impurity from mono-propylene glycol. The first distillation column 1 may be configured to operate at a temperature within the range of 140 - 200 °C and a pressure within the range of 0.3 - 1.0 bar.

Further both arrangements disclose a second distillation column 2 for separating diols that have a boiling point lower than the boiling point of mono-propylene glycol, from mono-propylene glycol. The second distillation column 2 may be configured to operate at a temperature within the range of 90 - 150 °C and a pressure within the range of 0.05 - 0.2 bar.

Fig. 2a discloses an arrangement wherein the first distillation column 1 is operationally arranged before the second distillation column 2.

Fig. 2b discloses an arrangement, wherein the first distillation column 1 is operationally arranged after the second distillation column 2.

In the embodiment disclosed in Fig. 2a, the mixture comprising bio-based diols may be fed into the first distillation column 1. The first distillation process to be carried out in the first distillation column, thus results in the organic impurity being separated from e.g. mono-propylene glycol. As a result of the distillation process in the first distillation column the organic impurity may be removed in a first bottom stream 1bs from the first distillation column 1 and the mono-propylene glycol may be removed in a first top stream 1ts from the first distillation column 1.

The first top stream 1ts may then be fed into the second distillation column 2 for separating diols that have a boiling point lower than mono-propylene glycol, from the mono-propylene glycol. As a result of the distillation process in the second distillation column diols that have a boiling point lower than the boiling point of mono-propylene glycol may be removed in a second top stream 2ts from the second distillation process 2 and mono-propylene glycol may be removed in a second bottom stream 2bs from the second distillation process 2.

In the embodiment disclosed in Fig. 2b, the first distillation column 1 is operationally arranged after the second distillation column 2. As a result of the distillation process in the second distillation column 2 diols that have a boiling point lower than the boiling point of mono-propylene glycol may be removed in a second top stream 2ts from the second distillation column 2 and mono-propylene glycol may be removed in a second bottom stream 2bs from the second distillation column 2. The second bottom stream 2bs may in addition to mono-propylene glycol comprise the organic impurity.

The second bottom stream 2bs may then be fed into the first distillation column 1. As a result of the used distillation conditions in the first distillation column, the possible azeotrope between mono-propylene glycol and the organic impurity may be broken allowing the mono-propylene glycol to be separated from organic impurity. As a result of the distillation process in the first distillation column 1 the organic impurity may be removed in a first bottom stream 1bs from the first distillation column 1 and mono-propylene glycol may be removed in a first top stream 1ts from the first distillation column 1.

### Example 1 - Distillation of mixtures comprising bio-derived diols

In this example, mixtures comprising bio-based diols were first subjected to the first distillation process, and then followed by the second distillation process.

The mixture of example 1a comprised:
90.1 weight-% of mono-propylene glycol (PG),
4.5 weight-% of H₂O,
4.5 weight-% of 2,3-butanediol (2,3-BTD),
0.9 weight-% of organic impurity

An otherwise corresponding example 1b was carried out but where the mixture did not comprise water. The mixture in example 1b comprised:
94.3 weight-% of mono-propylene glycol (PG),
4.7 weight-% of 2,3-butanediol (2,3-BTD),
0.9 weight-% of organic impurity

The temperatures and pressures used as well as the results for example 1a are presented in the below table:

| Top pressure in the first distillation column was p = 0,8 bar; and top pressure in the second distillation column was p = 0.1 bar. | | | | | | |
|---|---|---|---|---|---|---|
| | | | first distillation column | | second distillation column | |
| | Unit | FEED | first bottom stream | first top stream | second bottom stream | second top stream |
| Temperature | °C | 45 | | | | |
| Temperature, top of distillation column | °C | | | 174,2 | | 107,0 |
| Temperature, reboiler | °C | | 182,6 | | 133,4 | |
| Temperature, condenser | °C | | | 138,1 | | 56,5 |
| Pressure | bar | 4 | 0, 86 | 0,80 | 0, 15 | 0, 10 |
| Mass Flows | kg/hr | 1110 | 30 | 1080 | 850 | 230 |
| H2O | kg/hr | 50 | trace | 50 | trace | 50,0 |
| PG | kg/hr | 1000 | 23,0 | 977,0 | 845,2 | 131,8 |
| Organic impurity | kg/hr | 10 | 7,0 | 3,0 | 2,4 | 0,6 |
| 2,3-BTD | kg/hr | 50 | trace | 50 | 2,4 | 47,6 |
| Distillation stages | | | 100 | | 100 | |
| Reflux ratio | | | 15 | | 15 | |
| Reboiler duty | MW | | 4,27 | | 1,24 | |
| PG purity reached | % | | | | 99,4 | |

As can be seen from the above table a purity of 99.4 % of mono-propylene glycol was reached.

The temperatures and pressures used as well as the results for example 1b are presented in the below table:

| Top pressure in the first distillation column was p = 0,8 bar; and top pressure in the second distillation column was p = 0.1 bar. | | | | | | |
|---|---|---|---|---|---|---|
| | | | first distillation column | | second distillation column | |
| | Unit | FEED | first bottom stream | first top stream | second bottom stream | second top stream |
| Temperature | °C | 45 | | | | |
| Temperature, top of distillation column | °C | | | 179,6 | | 123,4 |
| Temperature, reboiler | °C | | 182, 6 | | 133,4 | |
| Temperature, condenser | °C | | | 179,5 | | 123,1 |
| Pressure | bar | 4 | 0,86 | 0,80 | 0,15 | 0,10 |
| Mass Flows | kg/hr | 1060 | 30 | 1030 | 850 | 180 |
| H2O | kg/hr | 0 | 0 | 0 | 0 | 0 |
| PG | kg/hr | 1000 | 23,8 | 976,2 | 846,4 | 129,9 |
| Organic impurity | kg/hr | 10 | 6,2 | 3,8 | 3,0 | 0,7 |
| 2,3-BTD | kg/hr | 50 | 0,0 | 50,0 | 0,6 | 49,4 |
| Distillation stages | | | 100 | | 100 | |
| Reflux ratio | | | 15 | | 45 | |
| Reboiler duty | MW | | 3,27 | | 1,65 | |
| PG purity reached | % | | | | 99,6 | |

As can be seen from the above table a purity of 99.6 % of mono-propylene glycol was reached.

### Example 2 - Distillation of mixtures comprising bio-derived diols

In this example, mixtures were subjected to the first distillation process after it had been subjected to the second distillation process.

The mixture of example 2a comprised:
90.1 weight-% of mono-propylene glycol (PG),
4.5 weight-% of H₂O,
4.5 weight-% of 2,3-butanediol (2,3-BTD),
0.9 weight-% of organic impurity

An otherwise corresponding example 2b was carried out but where the mixture did not comprise water. The mixture in example 2b comprised:
94.3 weight-% of mono-propylene glycol (PG),
4.7 weight-% of 2,3-butanediol (2,3-BTD),
0.9 weight-% of organic impurity

The temperatures and pressures used as well as the results for example 2a are presented in the below table:

| Top pressure in the first distillation column was p = 0,8 bar; and top pressure in the second distillation column was p = 0.1 bar. | | | | | | |
|---|---|---|---|---|---|---|
| | | | second distillation column | | first distillation column | |
| | Units | FEED | second bottom stream | second top stream | first bottom stream | first top stream |
| Temperature | °C | 45 | | | | |
| Temperature, top of distillation column | °C | | | 91,2 | | 179,9 |
| Temperature, reboiler | °C | | 135,0 | | 182,5 | |
| Temperature, condenser | °C | | | 48,5 | | 179,9 |
| Pressure | bar | 4 | 0,16 | 0,10 | 0,86 | 0,80 |
| Mass Flows | kg/hr | 1110 | 1000 | 110 | 40 | 960 |
| H2O | kg/hr | 50 | trace | 50,0 | 0 | 0 |
| PG | kg/hr | 1000 | 989,7 | 10,3 | 33,0 | 956,7 |
| Organic impurity | kg/hr | 10 | 10,0 | 0,0 | 7,0 | 2,9 |
| 2,3-BTD | kg/hr | 50 | 0,4 | 49,6 | trace | 0,4 |
| Distillation stages | | | 80 | | 100 | |
| Reflux ratio | | | 60 | | 20 | |
| Reboiler duty | MW | | 2,96 | | 3,91 | |
| PG purity reached | % | | | | | 99,7 |

As can be seen from the above table a purity of 99.7 % of mono-propylene glycol was reached.

The temperatures and pressures used as well as the results for example 2b are presented in the below table:

| Top pressure in the first distillation column was p = 0,8 bar; and top pressure in the second distillation column was p = 0.1 bar. | | | | | | |
|---|---|---|---|---|---|---|
| | | | second distillation column | | first distillation column | |
| | Units | FEED | second bottom stream | second top stream | first bottom stream | first top stream |
| Temperature | °C | 45 | | | | |
| Temperature, top of distillation column | °C | | | 121,7 | | 179,9 |
| Temperature, reboiler | °C | | 135, 0 | | 182,5 | |
| Temperature, condenser | °C | | | 121,5 | | 179,9 |
| Pressure | bar | 4 | 0, 16 | 0, 10 | 0, 86 | 0,80 |
| Mass Flows | kg/hr | 1060 | 1000 | 60 | 40 | 960 |
| H2O | kg/hr | 0 | 0 | 0 | 0 | 0 |
| PG | kg/hr | 1000 | 989,3 | 10,7 | 33,0 | 956,4 |
| Organic impurity | kg/hr | 10 | 10,0 | 0,0 | 7,0 | 2,9 |
| 2,3-BTD | kg/hr | 50 | 0,7 | 49,3 | trace | 0,7 |
| Distillation stages | | | 80 | | 100 | |
| Reflux ratio | | | 250 | | 20 | |
| Reboiler duty | MW | | 2,88 | | 3,91 | |
| PG purity reached | % | | | | | 99,6 |

As can be seen from the above table a purity of 99.6 % of mono-propylene glycol was reached.

### Example 3 - Distillation of a mixture comprising bio-derived diols

In this example, a mixture was subjected to the first distillation process after it had been subjected to the second distillation process.

The mixture of example 2a comprised:
88.5 weight-% of mono-polyethylene glycol (PG),
4.4 weight-% of H₂O,
2.7 weight-% of 2,3-butanediol (2,3-BTD),
4.4 weight-% of organic impurity

The temperatures and pressures used as well as the results for example 3 are presented in the below table:

| Top pressure in the first distillation column was p = 1.0 bar; and top pressure in the second distillation column was p = 0.1 bar. | | | | | | |
|---|---|---|---|---|---|---|
| | | | second distillation column | | first distillation column | |
| | Unit | FEED | second bottom | second top | second bottom | second top |
| | | | stream | stream | stream | stream |
| Temprature | °C | 45 | | | | |
| Temperature, top of distillation column | °C | | | 91,2 | | 187,2 |
| Temperature, reboiler | °C | | 135,0 | | 189,9 | |
| Temperature, condenser | °C | | | 48,5 | | 187,2 |
| Pressure | bar | 4 | 0,16 | 0,10 | 1,06 | 1,00 |
| Mass Flows | kg/hr | 1130 | 1020 | 110 | 80 | 940 |
| H2O | kg/hr | 50 | trace | 50,0 | 0 | 0 |
| PG | kg/hr | 1000 | 989,4 | 10,6 | 53,8 | 935,6 |
| Organic impurity | kg/hr | 30 | 29,9 | 0,1 | 26,2 | 3,7 |
| 2,3-BTD | kg/hr | 50 | 0,7 | 49,3 | trace | 0,7 |
| Distillation stages | | | 80 | | 100 | |
| Reflux ratio | | | 60 | | 25 | |
| Reboiler duty | MW | | 2,96 | | 3,91 | |
| PG purity reached | % | | | | | 99, 5 |

As can be seen from the above table a purity of 99.5 % of mono-propylene glycol was reached.

It is obvious to a person skilled in the art that with the advancement of technology, the basic idea may be implemented in various ways. The embodiments are thus not limited to the examples described above; instead they may vary within the scope of the claims.

It is understood that reference to 'an' item refers to one or more of those items. The term "comprising" is used in this specification to mean including the feature(s) or act(s) followed thereafter, without excluding the presence of one or more additional features or acts.

## Claims

1. A method for recovering mono-propylene glycol from a mixture comprising bio-derived diols and an organic impurity, wherein the mixture comprises mono-propylene glycol in an amount of at least 50 weight-% of the total weight of the mixture, wherein the organic impurity is **characterized by** a retention time of 6.5 - 6.7 minutes when determined by gas-chromatography-flame ionization detector (GC-FID) and by the tallest peak value at 59 m/z when determined by gas-chromatography-mass-spectrometer (GC-MS), and wherein the method comprises:
(ia) separating the organic impurity from mono-propylene glycol in a first distillation process, wherein the first distillation process is carried out at a temperature within the range of 140 - 200 °C and a pressure within the range of 0.3 - 1.0 bar; and
(ib) separating diols that have a boiling point lower than the boiling point of mono-propylene glycol, from mono-propylene glycol in a second distillation process, wherein the second distillation process is carried out at a temperature within the range of 90 - 150 °C and a pressure within the range of 0.05 - 0.2 bar;
(ii) recovering mono-propylene glycol, and wherein the method comprises:
- removing the organic impurity in a first bottom stream from the first distillation process (ia); and
- removing mono-propylene glycol in a first top stream from the first distillation process (ia).

2. The method of claim 1, wherein (ia) is carried out before (ib) or (ia) is carried out after (ib) .

3. The method of any one of claims 1 - 2, wherein the method comprises:
- removing diols that have a boiling point lower than the boiling point of mono-propylene glycol in a second top stream from the second distillation process (ib); and
- removing mono-propylene glycol in a second bottom stream from the second distillation process (ib) .

4. The method of any one of claims 1 - 3, wherein the first distillation process (ia) is carried out before the second distillation process (ib).

5. The method of claim 4, wherein the first top stream comprises, in addition to mono-propylene glycol, diols that have a boiling point lower than the boiling point of mono-propylene glycol.

6. The method of any one of claims 4 - 5, wherein the method comprises feeding the first top stream into the second distillation process.

7. The method of any one of claims 1 - 3, wherein the first distillation process (ia) is carried out after the second distillation process (ib).

8. The method of claim 7, wherein the second bottom stream comprises, in addition to mono-propylene glycol, the organic impurity.

9. The method of any one of claims 7 - 8, wherein the method comprises feeding the second bottom stream into the first distillation process.

10. The method of any one of the preceding claims, wherein the mixture comprises mono-ethylene glycol, mono-propylene glycol, butylene glycol, and an organic impurity in an amount of at least 80 weight-%, or at least 85 weight-%, or at least 90 weight-%, or at least 92 weight-%, of the total weight of the mixture.

11. The method of any one of the preceding claims, wherein the mixture comprises mono-propylene glycol in an amount of at least 60 weight-%, or at least 70 weight-%, or at least 80 weight-%, or at least 90 weight-%, of the total weight of the mixture.

12. The method of any one of the preceding claims, wherein the first distillation process is carried out at a temperature within the range of 150 - 190 °C, or 160 - 180 °C.

13. The method of any one of the preceding claims, wherein the first distillation process is carried out at a pressure within the range of 0.5 - 0.9 bar, or 0.7 - 0.8 bar.

14. The method of any one of the preceding claims, wherein the second distillation process is carried out at a temperature within the range of 100 - 140 °C, or 110 - 130 °C.

15. The method of any one of the preceding claims, wherein the second distillation process is carried out at a pressure within the range of 0.1 - 0.15 bar.

16. The method of any one of the preceding claims, wherein the method comprises recovering mono-propylene glycol at a concentration of at least 99 weight-%, or at least 99.5 weight-%, or at least 99.7 weight-%, or at least 99.9 weight-%, or at least 99.99 weight-%.

## Patentansprüche

1. Verfahren zur Rückgewinnung von Monopropylenglycol aus einem Gemisch, umfassend Diole biologischen Ursprungs und eine organische Verunreinigung, wobei das Gemisch Monopropylenglycol in einer Menge von mindestens 50 Gew.-% des Gesamtgewichts des Gemischs umfasst, wobei die organische Verunreinigung durch eine Retentionszeit von 6,5 - 6,7 Minuten, wenn mittels Gaschromatographie-Flammenionisationsdetektor (GC-FID) bestimmt wird, und durch den höchsten Peakwert bei 59 m/z, wenn mittels Gaschromatographie-Massenspektrometer (GC-MS) bestimmt wird, gekennzeichnet ist, und wobei das Verfahren umfasst:
(ia) das Abtrennen der organischen Verunreinigung von Monopropylenglycol in einem ersten Destillationsprozess, wobei der erste Destillationsprozess bei einer Temperatur im Bereich von 140 - 200 °C und einem Druck im Bereich von 0,3 - 1,0 bar durchgeführt wird; und
(ib) das Abtrennen von Diolen, die einen niedrigeren Siedepunkt als der Siedepunkt von Monopropylenglycol aufweisen, von Monopropylenglycol in einem zweiten Destillationsprozess, wobei der zweite Destillationsprozess bei einer Temperatur im Bereich von 90 - 150 °C und einem Druck im Bereich von 0,05 - 0,2 bar durchgeführt wird;
(ii) das Rückgewinnen von Monopropylenglycol, wobei das Verfahren umfasst:
- das Entfernen der organischen Verunreinigung in einem ersten Bodenstrom aus dem ersten Destillationsprozess (ia); und
- das Entfernen von Monopropylenglycol in einem ersten Kopfstrom aus dem ersten Destillationsprozess (ia).

2. Verfahren gemäß Anspruch 1, wobei (ia) vor (ib) durchgeführt wird oder (ia) nach (ib) durchgeführt wird.

3. Verfahren gemäß einem der Ansprüche 1 - 2, wobei das Verfahren umfasst:
- das Entfernen von Diolen, die einen niedrigeren Siedepunkt als der Siedepunkt von Monopropylenglycol aufweisen, in einem zweiten Kopfstrom aus dem zweiten Destillationsprozess (ib); und
- das Entfernen von Monopropylenglycol in einem zweiten Bodenstrom aus dem zweiten Destillationsprozess (ib).

4. Verfahren gemäß einem der Ansprüche 1 - 3, wobei der erste Destillationsprozess (ia) vor dem zweiten Destillationsprozess (ib) durchgeführt wird.

5. Verfahren gemäß Anspruch 4, wobei der erste Kopfstrom zusätzlich zu Monopropylenglycol Diole umfasst, die einen Siedepunkt aufweisen, der niedriger als der Siedepunkt von Monopropylenglycol ist.

6. Verfahren gemäß einem der Ansprüche 4 - 5, wobei das Verfahren das Zuführen des ersten Kopfstroms in den zweiten Destillationsprozess umfasst.

7. Verfahren gemäß einem der Ansprüche 1 - 3, wobei der erste Destillationsprozess (ia) nach dem zweiten Destillationsprozess (ib) durchgeführt wird.

8. Verfahren gemäß Anspruch 7, wobei der zweite Bodenstrom zusätzlich zu Monopropylenglycol die organische Verunreinigung umfasst.

9. Verfahren gemäß einem der Ansprüche 7 - 8, wobei das Verfahren das Zuführen des zweiten Bodenstroms in den ersten Destillationsprozess umfasst.

10. Verfahren gemäß einem der vorstehenden Ansprüche, wobei das Gemisch Monoethylenglycol, Monopropylenglycol, Butylenglycol und eine organische Verunreinigung in einer Menge von mindestens 80 Gew.-% oder mindestens 85 Gew.-% oder mindestens 90 Gew.-% oder mindestens 92 Gew.-% des Gesamtgewichts des Gemischs umfasst.

11. Verfahren gemäß einem der vorstehenden Ansprüche, wobei das Gemisch Monopropylenglycol in einer Menge von mindestens 60 Gew.-% oder mindestens 70 Gew.-% oder mindestens 80 Gew.-% oder mindestens 90 Gew.-% des Gesamtgewichts das Gemisch umfasst.

12. Verfahren gemäß einem der vorstehenden Ansprüche, wobei der erste Destillationsprozess bei einer Temperatur im Bereich von 150 - 190 °C oder 160 - 180 °C durchgeführt wird.

13. Verfahren gemäß einem der vorstehenden Ansprüche, wobei der erste Destillationsprozess bei einem Druck im Bereich von 0,5 - 0,9 bar oder 0,7 - 0,8 bar durchgeführt wird.

14. Verfahren gemäß einem der vorstehenden Ansprüche, wobei der zweite Destillationsprozess bei einer Temperatur im Bereich von 100 - 140 °C oder 110 - 130 °C durchgeführt wird.

15. Verfahren gemäß einem der vorstehenden Ansprüche, wobei der zweite Destillationsprozess bei einem Druck im Bereich von 0,1 - 0,15 bar durchgeführt wird.

16. Verfahren gemäß einem der vorstehenden Ansprüche, wobei das Verfahren die Rückgewinnung von Monopropylenglycol in einer Konzentration von mindestens 99 Gew.-% oder mindestens 99,5 Gew.-% oder mindestens 99,7 Gew.-% oder mindestens 99,9 Gew.-% oder mindestens 99,99 Gew.-% umfasst.

## Revendications

1. Procédé de récupération de mono-propylèneglycol à partir d'un mélange comprenant des diols d'origine biologique et une impureté organique, dans lequel le mélange comprend du mono-propylèneglycol en une quantité d'au moins 50 % en poids du poids total du mélange, dans lequel l'impureté organique est **caractérisée par** un temps de rétention compris entre 6,5 et 6,7 minutes lorsque déterminé par chromatographie en phase gazeuse avec détecteur à ionisation de flamme (GC-FID) et par la valeur de pic la plus élevée à 59 m/z lorsque déterminée par chromatographie en phase gazeuse couplée à la spectrométrie de masse (GC-MS), et dans lequel le procédé comprend :
(ia) séparer l'impureté organique du mono-propylèneglycol lors d'un premier processus de distillation, dans lequel le premier processus de distillation est exécuté à une température comprise dans la plage de 140 à 200 °C et à une pression comprise dans la plage de 0,3 à 1,0 bar ; et
(ib) séparer du mono-propylèneglycol les diols qui ont un point d'ébullition inférieur au point d'ébullition du mono-propylèneglycol, lors d'un second processus de distillation, dans lequel le second processus de distillation est exécuté à une température comprise dans la plage de 90 à 150 °C et à une pression comprise dans la plage de 0,05 à 0,2 bar ;
(ii) récupérer le mono-propylèneglycol, et dans lequel le procédé comprend :
- retirer l'impureté organique dans un premier effluent de fond issu du premier processus de distillation (ia) ; et
- retirer le mono-propylèneglycol dans un premier effluent de tête issu du premier processus de distillation (ia).

2. Procédé selon la revendication 1, dans lequel (ia) est exécutée avant (ib), ou (ia) est exécutée après (ib).

3. Procédé selon l'une ou l'autre des revendications 1 et 2, dans lequel le procédé comprend :
- retirer les diols qui ont un point d'ébullition inférieur au point d'ébullition du mono-propylèneglycol dans un second effluent de tête issu du second processus de distillation (ib) ; et
- retirer le mono-propylèneglycol dans un second effluent de fond issu du second processus de distillation (ib).

4. Procédé selon l'une quelconque des revendications 1 à 3, dans lequel le premier processus de distillation (ia) est exécuté avant le second processus de distillation (ib).

5. Procédé selon la revendication 4, dans lequel le premier effluent de tête comprend, en plus du mono-propylèneglycol, des diols qui ont un point d'ébullition inférieur au point d'ébullition du mono-propylèneglycol.

6. Procédé selon l'une ou l'autre des revendications 4 et 5, dans lequel le procédé comprend amener le premier effluent de tête à subir le second processus de distillation.

7. Procédé selon l'une quelconque des revendications 1 à 3, dans lequel le premier processus de distillation (ia) est exécuté après le second processus de distillation (ib).

8. Procédé selon la revendication 7, dans lequel le second effluent de fond comprend, en plus du mono-propylèneglycol, l'impureté organique.

9. Procédé selon l'une ou l'autre des revendications 7 et 8, dans lequel le procédé comprend amener le second effluent de fond à subir le premier processus de distillation.

10. Procédé selon l'une quelconque des revendications précédentes, dans lequel le mélange comprend du mono-éthylèneglycol, du mono-propylèneglycol, du butylène glycol et une impureté organique en une quantité d'au moins 80 % en poids, ou d'au moins 85 % en poids, ou d'au moins 90 % en poids, ou d'au moins 92 % en poids, du poids total du mélange.

11. Procédé selon l'une quelconque des revendications précédentes, dans lequel le mélange comprend du mono-propylèneglycol en une quantité d'au moins 60 % en poids, ou d'au moins 70 % en poids, ou d'au moins 80 % en poids, ou d'au moins 90 % en poids, du poids total du mélange.

12. Procédé selon l'une quelconque des revendications précédentes, dans lequel le premier processus de distillation est exécuté à une température comprise dans la plage de 150 à 190 °C, ou de 160 à 180 °C.

13. Procédé selon l'une quelconque des revendications précédentes, dans lequel le premier processus de distillation est exécuté à une pression comprise dans la plage de 0,5 à 0,9 bar, ou de 0,7 à 0,8 bar.

14. Procédé selon l'une quelconque des revendications précédentes, dans lequel le second processus de distillation est exécuté à une température comprise dans la plage de 100 à 140 °C, ou de 110 à 130 °C.

15. Procédé selon l'une quelconque des revendications précédentes, dans lequel le second processus de distillation est exécuté à une pression comprise dans la plage de 0,1 à 0,15 bar.

16. Procédé selon l'une quelconque des revendications précédentes, dans lequel le procédé comprend récupérer le mono-propylèneglycol à une concentration d'au moins 99 % en poids, ou d'au moins 99,5 % en poids, ou d'au moins 99,7 % en poids, ou d'au moins 99,9 % en poids, ou d'au moins 99,99 % en poids.
